Europäisches Patentamt

European Patent Office

Office européen des brevets

(19)

(11) Publication number: **0 225 329 B1**

# EUROPEAN PATENT SPECIFICATION

(12)

(45) Date of publication of patent specification: **12.08.92**

(51) Int. Cl.⁵: **A61K 39/102**, A61K 39/085, A61K 39/09, A61K 39/104

(21) Application number: **86901755.8**

(22) Date of filing: **17.03.86**

(86) International application number: **PCT/AU86/00071**

(87) International publication number: **WO 86/05691 (09.10.86 86/22)**

(54) **NON-ADJUVENATED VACCINE.**

(30) Priority: **27.03.85 AU 9928/85**
**15.08.85 AU 1968/85**

(43) Date of publication of application: **16.06.87 Bulletin 87/25**

(45) Publication of the grant of the patent: **12.08.92 Bulletin 92/33**

(84) Designated Contracting States: **BE CH DE FR GB IT LI NL SE**

(56) References cited:

BIOLOGICAL ABSTRACTS/RRM, no. 23043018 J. SEIFERT et al.: "Oral application of bacterial vaccine for infection prophylaxis"

BIOLOGICAL ABSTRACTS, vol. 65, no. 2, 1978, no. 894, ref. 9268 J.R. GERKE et al.: "Oral vaccination and multivalent vaccine against Pseudomonas aeruginosa keratitis"

(73) Proprietor: **RHONE-POULENC AUSTRALIA PTY. LIMITED**
**19-29 Paramount Road**
**West Footscray, Victoria 3012(AU)**

(72) Inventor: **CLANCY, Robert Llewellyn**
**99 Carington Parade New Lambton**
**Newcastle NSW 2305(AU)**

(74) Representative: **Brown, John David et al**
**FORRESTER & BOEHMERT Franz-Joseph-Strasse 38**
**W-8000 München 40(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

INFECTION AND IMMUNITY, vol. 39, no.2, February 1983, pages 491-496 R.L. CLANCY et al.: "Specific immune response in the respiratory tract after administration of an oral polyvalent bacterial vaccine"

Japan Journal of Antibiotics, 38(9) 1985 2444-2452 M. Sugita et al.: "Fundamental and Clinical Studies of S-6472 Sustained Release Preparation of Cefaclor in the Pedriatic Field" (BIOSIS English language abstract 81060342)

## Description

Technical Field

The present invention relates to an enteral monobacterial vaccine comprising killed Haemophilus influenza or Streptococcus pneumoniae, a process for the manufacture of such a vaccine and a method of preventing acute mucosal infections in humans having chronic mucosal disease by administering such a vaccine.

Background Art

Acute episodes of bronchitis in cigarette smokers are a major health problem and are in particular a cause of morbidity and mortality with patients with chronic obstructive lung disease (COLD). The upper respiratory tract of these patients is commonly colonised with Haemophilus influenzae and/or Streptococcus pneumoniae, and it has been held that a shift in the host-bacteria balance favouring infection with these organisms is the immediate cause of acute bronchitis. Children with cystic fibrosis commonly suffer from chronic infection by Pseudomonas aeruginosa and Staphylococcus aureus.

Polybacterial vaccines containing a selection of killed bacteria normally associated with infection of the respiratory tract have been available for many years. The polybacterial vaccines contain an adjuvant of conventional type whose function according to established methodology is to enhance the response of the patient to the antigen thereby leading to enhanced immunity. The adjuvant may be a chemical agent. Alternatively, the variety of organisms themselves function in a non-specific way to activate the immune system. Such adjuvant-containing polybacterial vaccine has been commercially available in tablet form for oral digestion. The tablets are enteric coated to allow passage through the stomach, followed by stimulation of gut-associated lymphoid tissue. However, the effectiveness of these polybacterial vaccines has been disappointing in the treatment of acute bacterial infections of the respiratory tract in humans having chronic mucosal inflammations.

Clancy et al (1983) Infection and Immunity, Vol.39, No.2, p491-496, discloses the use of oral polyvalent vaccines obtaining a specific immune response in the saliva of normal individuals.

Gerke et al (1977) Invest. Opthalmol. Visual Sci., Vol16, No.1, p76-80 discloses vaccination of mice against P. aeruginosa infection of the cornea.

It is an object of the present invention to provide an enteral vaccine against infections of mucosal surfaces in humans having long term mucosal disease.

Disclosure of Invention

In a first aspect, the present invention provides an enteral monobacterial vaccine comprising killed bacteria for immunisation against bacterial infection of mucosal sites. Examples for such bacteria are Haemophilus influenzae, Streptococcus pneumoniae, Pseudomonas aeruginosa and Staphylococcus aureus. Preferred is a vaccine comprising killed Haemophilus influenzae. A particular aspect of the vaccine of the present invention is that no adjuvant is added to the killed bacteria, i.e. that the vaccine stimulates only a limited antigenic response. Adjuvants usually added to vaccines and absent in the vaccine of the present invention are killed bacteria known to illicit a strong antigenic response, e.g. killed Mycobacterium tuberculosis such as Bacillus Calmette-Guerin (BCG), or killed Corynebacterium parvum, a selection of closely related bacteria, whereby each species acts as an adjuvant for the other bacteria, or inorganic polymeric material, e.g. aluminium oxide or aluminium phosphate, which is useful especially in parenteral vaccines.

The enteric vaccine may be in the form of tablets, especially enteric coated tablets, granules, capsules or dragees for oral administration, or provided e.g. as suppositories for rectal administration. The dosage unit form may contain from approximately $10^9$ bacteria to approximately $10^{13}$ bacteria, preferably from approximately $10^{10}$ bacteria to approximately $10^{12}$ bacteria, together with suitable carriers of organic or inorganic nature.

Suitable carriers are especially fillers, such as sugars, for example lactose, saccharose, mannitol or sorbitol, cellulose preparations and/or calcium phosphates, for example tricalcium phosphate or calcium hydrogen phosphate, amino acids, for example glycine, also binders, such as starch pastes using, for example, corn, wheat, rice or potato starch, gelatine, tragacanth, methylcellulose, hydroxypropylmethylcellulose, sodium carboxymethylcellulose and/or polyvinylpyrrolidone, and/or, if desired, disintegrators, such as the above-mentioned starches, also carboxymethyl starch, crosslinked polyvinylpyrrolidone, agar, alginic

EP 0 225 329 B1

acid or a salt thereof, such as sodium alginate. Adjuncts are especially flow-regulating agents and lubricants, for example silica, talc, stearic acid or salts thereof, such as magnesium stearate or calcium stearate, and/or polyethylene glycol, further choleretic agents, e.g. sodium taurocholate, sodium tauroglycocholate or ox bile. Dragée cores are provided with suitable coatings that may be resistant to gastric juices, there being used, inter alia, concentrated sugar solutions which may contain gum arabic, talc, polyvinylpyrrolidone, polyethylene glycol and/or titanium dioxide, lacquer solutions in suitable organic solvents or solvent mixtures or, for the production of coatings that are resistant to gastric juices, solutions of suitable cellulose preparations, such as acetylcellulose phthalate or hydroxypropylmethylcellulose phthalate. Dyes or pigments can be added to the tablets or dragée coatings, for example for identification purposes or to indicate different doses of active ingredient. Compounds illiciting an antigenic response, i.e. adjuvants, are excluded as carriers or adjuncts in the vaccine of the invention.

In a second aspect, the present invention relates to processes known per se for the manufacture of the inventive vaccine, characterised in that the bacteria are cultured, then killed, lyophilised and/or dried, mixed with carriers, fillers and adjuncts, and brought into the form of a therapeutically applicable enteric pharmaceutical composition.

Such processes for the manufacture of vaccines are known in the art. The bacteria may be grown on plates, e.g. agar plates containing various nutrients, for example blood or "chocolate" agar plates, or in suspension in fermentation broth containing nutrients in dissolved form, e.g. milk hydrolysates, lactalbumin hydrolysates, corn steep liquors, glucose, starches, tryptic soy broth and the like, and growth stimulating substances, e.g. hormones and coenzymes, also in the form of serum dilutions, for example of horse serum or foetal calf serum. The cultures must be kept under strictly aseptic conditions, and optionally small amounts of antibiotics, e.g. bacitracin, are added to prevent overgrow by unwanted bacteria.

The organisms are killed for example with formalin, phenol or ether, optionally homogenised and washed extensively. Sterility of the killed bacteria has to be tested carefully, e.g. by inoculation into a medium or an agar plate known to allow rapid growth of viable bacteria. Pharmaceutical preparations for oral or rectal administration are obtained from the killed bacteria by conventional lyophilising, drying, mixing, granulating and/or confectioning processes under sterile conditions.

According to a third aspect of the present invention there is provided use of killed bacteria for the manufacture of an enteral non-adjuvenated monobacterial vaccine for immunisation against bacterial infection of mucosal sites.

There is disclosed a method of preventing acute mucosal infections in humans having chronic mucosal disease, characterised in that a vaccine of the invention is administered to a patient in need thereof. Preferably one to three unit doses of the vaccine containing $10^{10}$ to $10^{12}$ killed bacteria are administered for two to five consecutive days. More than one course may be required, and three courses at approximately three to five weeks intervals are preferred.

The invention is applicable to patients having long term diseases of mucosal sites, e.g. of the respiratory tract, eye, urogenital system and the gut. These mucosal sites form part of a common mucosal system linked by an intermucosal cell traffic. Certain infections are restricted to mucosal sites and are thus not susceptible to antibodies in the bloodstream. In the present invention the antigen is administered enterally, i.e. to the gut, and activated cells then circulate through the blood to lodge at distant mucosal sites, where they secrete their antibody on the mucosal surface or act directly on it.

The vaccine of the present invention is particularly effective to provide immunity against acute bacterial infections of the respiratory tract, especially of the bronchi, under circumstances of chronic inflammation. Long term lung diseases are prevalent in patients who are smokers or ex-smokers. This may include patients with chronic lung diseases, such as chronic bronchitis and other diseases associated with smoking, and children suffering from cystic fibrosis-related lung diseases.

Surprisingly, it appears that the immunisation efficacy of the vaccine arises from the absence of adjuvants, which would normally be included in such a vaccine formulation to promote the protective effect. The reason for this is not well understood, but may derive from the delicate balance which exists within the mucosa between suppressor and helper mechanisms in the production of immunity. The production of antibodies by B-cells is regulated by T-cells comprising helper and suppressor cells, though their exact functioning in the mucosal system is not understood. In patients with chronical inflamed bronchi (e.g. chronic bronchitis) there may be a dominance of suppressor T-cells such that the stimulation effect or the adjuvant is to produce a net suppression (rather than the intended activation) of the immune system. In the absence of adjuvant, a limited response occurs which is insufficient to trigger the suppressor mechanism. This is merely a hypothesis, but it is surprisingly found that for patients with chronic bronchitis, adddition of extra bacteria to the H. influenzae vaccine creates a vaccine which fails to protect against acute bronchitis.

4

Exemplory Mode of Carrying out Invention

The efficacy of the vaccine of the invention is shown by the results of a double-blind, prospective, placebo-controlled trial on sixty-seven patients with established chronic obstructive lung disease. No patient was taking steroids or immunosuppressive agents, but many took bronchodilator drugs and antibiotics as necessary.

Four groups were tested: Two placebo groups, one took glucose tablets, the second enteric coated tablets compounded in an identical fashion to the monobacterial vaccine of the invention and the state of the art polybacterial vaccine. The third group took a killed polybacterial vaccine representing the nearest state of the art and known to be effective in the prophylaxis of bacterial infections of the respiratory tract in normal, healthy humans. Each enteric coated tablet contained $1.5 \times 10^9$ H. influenzae, $10^9$ Staphylococcus aureus, $10^9$ of each of S. pneumoniae types I, II and III, $10^9$ Streptococcus, and 25 mg Fel Bovis sicc. (ox bile). These tablets are produced by Swiss Serum (Berne), and sold as Buccalin Berna®. The fourth group took a vaccine of the invention, namely enteric coated tablets each containing $10^{11}$ killed H. influenzae as described in the accompanying Example.

At zero time, each patient was assessed by a chest physician. A standard and comprehensive questionnaire was completed. In addition lung function was assessed by spirometry, and throat and sputum cultures were taken.

For every acute upper or lower respiratory tract infection, an "infection questionnaire" was completed, and sputum was taken for culture. Acute bronchitic episodes were defined as an increase in volume and purulence of sputum, usually associated with an increase in breathlessness, fever, and antibiotic therapy.

Three courses of tablets were given at 0, 28 and 56 days. For both placebos and the monobacterial vaccine of the invention, a course consisted of two tablets taken before breakfast each day for 3 consecutive days. For the polybacterial vaccine, each course was given according to the manufacturer's instructions, i.e. 1 tablet day 1, 2 tablets day 2, and 4 tablets day 3. At the completion of the study spirometry was repeated. The only patients lost from the study were those who died during the trial (Table 1).

Results for the 84 day study period are recorded in Table 2. No significant difference in the incidence of acute upper respiratory tract infection is detected between the 4 groups. However, a significant reduction in both the number of subjects with episode(s) of acute bronchitis (lower respiratory tract infection, P <0.005) and the absolute number of acute bronchitic episodes (P <0.002) is recorded in those subjects given the killed H. influenzae vaccine of the invention. The killed oral H. influenzae vaccine gave a protection rate of 90 % when compared with the placebo groups, while the polyvalent vaccine gave no protection.

H. influenzae was isolated from 69 % of the sputum samples taken at times of acute bronchitis, compared with an 8 % isolation rate for S. pneumoniae. Bacteriology was performed as usual: Throat swabs and sputum were directly inoculated onto blood agar and chocolate agar plates. H. influenzae isolates were identified by Gram stain, colony morphology in chocolate agar, "satellitism" along a staphylococcus streak on a blood agar plate and dependence of growth on X and V factors. S. pneumoniae isolates were identified by Gram stain, colony morphology and alpha heamolysis on a blood agar plate and optochin sensitivity.

There is no significant correlation between bacteria carriage rate and development of eposides of acute bronchitis. After four weeks of the study colonisation was similar for the subjects taking the placebo and the polybacterial vaccine, but less for the patients treated with the monobacterial vaccine, though the difference did not reach significance. There was a fall in colonisation rate through the following two months in the placebo groups of 28 %, the polybacterial vaccine group of 39 %, and the monobacterial vaccine group of 56 %. However, the trend towards a greater fall in the colonisation rate of the monobacterial vaccine group does not reach significance.

No difference in the pattern of antibody levels could be determined between the four groups. It is surprising that there is no enhancement of saliva antibodies in the case of the polyvalent vaccine, since an increase is known to occur in normal healthy adults. This supports the view that there is a difference in the immune systems of normal healthy patients and those with long term mucosal disease.

EP 0 225 329 B1

Table 1: Patient details

| | No. | Age | Sex male/ female | Smoking | | Died during trial | Hospitalised with bronchitis | Forced expiratory volume in one second | |
| | | | | Never | Current | | | Start of trial | End of trial |
|---|---|---|---|---|---|---|---|---|---|
| Placebo 1 | 16 | 65.5 ± 2.9 | 10/6 | 3 | 2 | 0 | 2 | 0.83 | 0.81 |
| Placebo 2 | 17 | 62 ± 2.2 | 16/1 | 2 | 1 | 2 | 5 | 0.94 | 0.99 |
| Polybacterial vaccine Buccalin Berna® | 17 | 66 ± 1.8 | 15/2 | 2 | 3 | 2 | 1 | 1.05 | 0.93 |
| Monobacterial vaccine of the invention | 17 | 64.7 ±1.64 | 14/3 | 1 | 2 | 0 | 0 | 1.01 | 1.01 |

Table 2: Protection induced by oral killed bacterial vaccines

| | Number of patients with acute infections of: | | Total number of acute lower respiratory tract infections | Isolation of H. influenzae in acute lower respiratory tract infections |
|---|---|---|---|---|
| | Upper respiratory tract | Lower respiratory tract | | |
| Placebo 1 | 2/16 | 9/16 | 13 | 12/13 |
| Placebo 2 | 0/17 | 11/17 | 23 | 13/23 |
| Polybacterial vaccine Buccalin Berna® | 6/17 | 9/17 | 13 | 11/13 |
| Monobacterial vaccine of the invention | 2/17 | 1/17 | 1 | 1/1 |

Example: Preparation of a vaccine containing H. influenzae Haemophilus influenzae type B No. 7279 (NIH 1946) is cultured in suspension in a medium of casamino acids essentially as described by P. Anderson, J. Pitt and D.H. Smith, Infection and Immunity 13, 581-589 (1976). The medium consists of 1 % Casamino Acids (Difco), 0.5 % yeast extract (Difco), 0.05 M sodium phosphate buffer and 0.5 % glucose, supplemented with horse blood (dilution 1:1000) and 1 µg/ml nicotinamide adenine dinucleotide (Sigma).

This yields approximately $1.3 \times 10^9$ colony-forming units per ml (CFU/ml) and $8 \times 10^{13}$ total viable cells. Similar results are obtained in Eugon broth in place of casamino acids ($7 \times 10^8$ CFU/ml). Cultures are shown to be pure by testing with an api 20B identification kit as well as by microscopic examination. The organism is notoriously pleomorphic in liquid culture.

Cells are harvested by continuous centrifugation, washed three times using physiological saline and inactivated by resuspending in 0.5 %, formalin in saline at a concentration of $5 \times 10^9$ CFU/ml. After 3 days, sterility is tested by inoculation into growth media and on CHB agar. No growth of any organism occurs after 7 days incubation at 36°C. Inactivated cells are lyophilised using a protective medium composed of dextran 40/lactose. In the absence of the protective medium, extensive cell disruption is observed. All precautions are taken during lyophilisation to maintain sterility of the product. The dispensing area, including the freeze drying chamber, are fumigated. Sterile trays, jars and other equipment are used and sterile air is introduced into the chamber at the end of the drying cycle. The weight of $8 \times 10^{13}$ cells lyophilised by this method amounts to 75.8 g ($10^{11}$ cells per 0.1 g).

Tablets are prepared from the lyophilised killed <u>Haemophilus influenzae</u> according to the following formula:

| | | |
|---|---|---|
| <u>granulation:</u> | lyophilised bacteria | 100 mg |
| | sodium taurocholate | 25 mg |
| | lactose | 100 mg |
| <u>granulating agent:</u> | alcohol 95SG type F3 | qs |
| <u>dry blend:</u> | maize starch | 10 g |
| | Polyplasdone XL™ | 7.5 mg |
| | magnesium stearate | 4 mg |

The compression weight is 246.5 mg, showing a core hardness of 5 kg (Monsanto) and a desintegration time of the core in pH 6.8 phosphate buffer of 14 minutes.

The tablets are coated with a coating solution consisting of:

| | |
|---|---|
| cellulose acetate phthalate | 12 g |
| propylene glycol | 3 g |
| Tween 80® | 1 g |
| alcohol abs. | 40 ml |
| iron oxide red pigment | 0.30 g |
| iron oxide yellow pigment | 1.20 g |
| acetone to | 100 ml |

The enteric coated tablet weighs 265 mg and shows a desintegration time of more than 2 hours in 0.1 N HCl and of 16 minutes in pH 6.8 phosphate buffer.

<u>Industrial Applicability</u>

The vaccine is able to provide improved immunity against acute bacterial infections of the respiratory tract, especially of the bronchi, under circumstances of chronic inflammation; and is particularly valuable for smokers or ex-smokers.

**Claims**

1. An enteral non-adjuvenated monobacterial vaccine comprising killed bacteria for immunisation against bacterial infection of mucosal sites and optionally a pharmaceutical carrier.

2. A vaccine according to Claim 1 comprising killed <u>Haemophilus influenzae</u>, <u>Streptococcus pneumoniae</u>, <u>Pseudomonas aeruginosa</u> or <u>Staphylococcus aureus</u>.

3. A vaccine according to Claim 1 or 2 in the form of enteric coated tables, granules, capsules or dragees for oral administration.

**4.** A vaccine according to Claim 1 or 2 in the form of suppositories for rectal administration.

**5.** A vaccine according to Claim 1 in unit dose form and comprising $10^{10}$ to $10^{12}$ bacteria per unit dose.

**6.** A vaccine according to any one of the preceding claims for immunisation against humans having chronic mucosal disease.

**7.** A vaccine according to Claim 6 for immunisation against bacterial infections of the respiratory tract.

**8.** A vaccine according to Claim 7 for immunisation against acute lower respiratory tract bacterial infections in patients with chronic obstructive lung disease.

**9.** A vaccine according to Claim 6, 7 or 8, for immunisation by administration of one to three unit doses of the vaccine containing $10^{10}$ to $12^{12}$ killed bacteria for two to five consecutive days and optional repetition.

**10.** A vaccine according to Claim 9, for immunisation wherein the administration course is repeated twice after a three to five week interval.

**11.** Use of killed bacteria for the manufacture of an enteral non-adjuvenated monobacterial vaccine for immunisation against bacterial infection of mucosal sites.

**Revendications**

**1.** Un vaccin monobactérien entérique sans adjuvant comprenant des bactéries tuées pour l'immunisation contre une infection bactérienne des sites muqueux et éventuellement, un support pharmaceutique.

**2.** Un vaccin suivant la revendication 1, comprenant des Haemophilus influenzae, Streptococcus pneumoniae, Pseudomonas aeruginosa ou Staphylococcus aureus tués.

**3.** Un vaccin suivant les revendications 1 ou 2 sous la forme de comprimés à revêtement entérique, granules, gélules ou dragées pour l'administration orale.

**4.** Un vaccin suivant les revendications 1 ou 2 sous la forme de suppositoires pour l'administration rectale.

**5.** Un vaccin suivant la revendication 1 sous forme de dose unitaire et comprenant $10^{10}$ à $10^{12}$ bactéries par dose unitaire.

**6.** Un vaccin suivant l'une quelconque des revendications précédentes pour l'immunisation des hommes contre une maladie chronique des muqueuses.

**7.** Un vaccin suivant la revendication 6 pour l'immunisation contre des infections bactériennes des voies respiratoires.

**8.** Un vaccin suivant la revendication 7 pour l'immunisation contre des infections bactériennes aiguës des voies respiratoires inférieures chez des patients ayant une maladie pulmonaire obstructive chronique.

**9.** Un vaccin suivant l'une quelconque des revendications 6, 7 ou 8 pour l'immunisation par administration d'une à trois doses unitaires du vaccin contenant $10^{10}$ à $10^{12}$ bactéries tuées pendant 2 à 5 jours consécutifs et répétition facultative.

**10.** Un vaccin suivant la revendication 9 pour l'immunisation dans lequel la série d'administration est répétée deux fois après un intervalle de 3 à 5 semaines.

**11.** Utilisation des bactéries tuées pour la fabrication d'un vaccin monobactérien entérique sans adjuvant pour l'immunisation contre une infection bactérienne des sites muqueux.

**Patentansprüche**

1. Enteraler monobakterieller Impfstoff ohne Hilfsmittel, der abgetötete Bakterien zur Immunisierung gegen bakterielle Infektion von Schleimhautstellen und ggf. einen pharmazeutischen Trägerstoff umfaßt.

2. Impfstoff nach Anspruch 1, gekennzeichnet durch abgetötete Haemophilus influenzae, Streptococcus pneumoniae, Pseudomonas aeruginosa oder Staphylococcus aureus.

3. Impfstoff nach Anspruch 1 oder 2 in Form von Tabletten mit Schutzhülle, Granulatkörnchen, Kapseln oder Dragees zur oralen Verabreichung.

4. Impfstoff nach Anspruch 1 oder 2 in Form von Suppositorien zur rektalen Verabreichung.

5. Impfstoff nach Anspruch 1 in Einheitsdosisform und $10^{10}$ bis $10^{12}$ Bakterien pro Einheitsdosis umfassend.

6. Impfstoff nach einem der vorangehenden Ansprüche zur Immunisierung gegen Menschen mit chronischer Schleimhauterkrankung.

7. Impfstoff nach Anspruch 6 zur Immunisierung gegen bakterielle Infektionen der Atemwege.

8. Impfstoff nach Anspruch 7 zur Immunisierung gegen akute bakterielle Infektionen der unteren Atemwege bei Patienten mit chronischer obstruktiver Lungenerkrankung.

9. Impfstoff nach Anspruch 6, 7 oder 8 zur Immunisierung durch Verabreichung von ein bis drei Einheitsdosen des Impfstoffes, enthaltend $10^{10}$ bis $10^{12}$ abgetötete Bakterien, über zwei bis fünf aufeinanderfolgende Tage und ggf. Wiederholung.

10. Impfstoff nach Anspruch 9 zur Immunisierung, wobei der Verabreichungsablauf zweimal nach einem drei bis fünf wöchigen Intervall wiederholt wird.

11. Verwendung abgetöteter Bakterien zur Herstellung eines enteralen monobakteriellen Impfstoffes ohne Hilfsmittel zur Immunisierung gegen bakterielle Infektion von Schleimhautstellen.